(19) Europäisches Patentamt
European Patent Office
Office européen des brevets

(11) **EP 3 785 687 B1**

(12) **EUROPEAN PATENT SPECIFICATION**

(45) Date of publication and mention
of the grant of the patent:
**21.06.2023 Bulletin 2023/25**

(21) Application number: **19193973.5**

(22) Date of filing: **28.08.2019**

(51) International Patent Classification (IPC):
**A61H 1/02** *(2006.01)*   **A61B 5/11** *(2006.01)*

(52) Cooperative Patent Classification (CPC):
**A61H 1/0259; A61H 1/0262;** A61B 5/112;
A61H 2201/1207; A61H 2201/1635;
A61H 2201/1642; A61H 2201/1664;
A61H 2201/5064; A61H 2201/5071;
A61H 2201/5092

(54) **GAIT TRAINING MACHINE AND METHOD OF USING SAME**

GANGTRAINER UND VERFAHREN ZUR VERWENDUNG DAVON

MACHINE D'ENTRAÎNEMENT À LA MARCHE ET SON PROCÉDÉ D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.03.2021 Bulletin 2021/09**

(73) Proprietor: **Hiwin Technologies Corp.
Taichung City 40852 (TW)**

(72) Inventors:
• **HUANG, CHAO-JU
  TAICHUNG CITY 40852 (TW)**
• **SHIU, JIA-MING
  TAICHUNG CITY 40852 (TW)**
• **WU, YI-JING
  TAICHUNG CITY 40852 (TW)**

(74) Representative: **Straus, Alexander et al
2K Patent- und Rechtsanwälte - München
Keltenring 9
82041 Oberhaching (DE)**

(56) References cited:
**WO-A1-2007/058512     JP-A- 2018 102 842
US-B1- 10 022 587**

EP 3 785 687 B1

**Description**

**BACKGROUND OF THE INVENTION**

**1. Field of the Invention**

The present invention relates to gait training machinery, in particular to a gait training machine.

**2. Description of the Related Art**

With the rise of the health awareness of modem people, a variety of training equipment about physical function has also arisen. In terms of gait training, in addition to the treadmill with running function, it also began to focus on the training of healthy gait posture.

When an ordinary person walks, a gait cycle begins from the right heel to the ground to left toe off the ground, then, the left heel to the ground to the right toe off the ground and finally back to the right heel to the ground. It can be seen that the left and right feet will alternately change during the entire gait cycle, and the ankle joints of the left and right feet have different actuation angles at different times. Among them, during the maximum stride in the entire gait cycle, the ankle joint movement angle of the left and right feet is the largest.

Regarding the angle of action of the ankle joint, it is necessary to know that the angle of the ankle joint is different during the period of the asynchronous state. From the initial contact to the midstance, the ankle joint exhibits a dorsiflexion state. During the period from toe-off to mid-swing of the contralateral foot, the ankle join exhibits plantar flexion. This shows that the angle of the ankle joint is cyclic when the person is walking.

In addition, the body's center-of-gravity will also have a shift between the feet during the gait cycle. During the midstance, the center-of-gravity of the body will fall on the support foot and thus bear most of the weight of the body. By mid-swing, the center-of-gravity of the body is transferred to the opposite foot, and the weight of the body is absorbed by the opposite foot. It follows this cycle.

From the above, it is not difficult to understand that if you want to train healthy gait posture, you need to let the user know the movement of the ankle joint and the center-of-gravity between the feet to adjust the healthy gait posture.

At present, the related technical materials include the US7266424B2 patent, which incorporates one or more pressure sensors into the biped robot to capture state changes and information collection during the gait cycle, thereby controlling the joint drive unit to prevent falls and other actions. The processor can perform calculations to obtain treadmill belt speed, foot impact time and left/right foot indication. The processor performs input data calculations from the sensors (162) and obtains calculations related to gait parameters. In addition, the system provides recommendations for gait correction. For example, the system can instruct the user to change the direction of their feet, to rotate their ankles, to bend their knees more, or to correct or to modify the patient's gait based on other adjustments to the analysis of the patient's gait data. Further, JP 2018102842 A discloses a walking training device with a simplified structure and easily evaluates training of walking of a person, comprising a step base, movable in a cross direction, on which a left foot of a trainer may be mounted, a step base, movable in a cross direction, on which a right foot of the trainer may be mounted, and pressure sensors for detecting pressure by a heel side and by toe side of left and right foot, and a microcomputer for evaluating training on the basis of the pressure detected. WO 2007058512 A1 discloses a gait trainer comprises a display, pedals, a control unit receiving physical information about the physical condition of the user while the pedals are moving, which defines an optimal exercise prescription based on the physical information, and providing exercise prescription to the display. The control unit accesses a server of a health-related organization by network, and defines the exercise prescription from the server. US 10022587 B1 discloses a walking trainer including a linear transmission unit. The pedals of the pedal units can be moved with carriages linearly and alternatively back and forth, on the other hand, and can also be moved with the associating top blocks up and down and biased with the respective rotary shafts relative to the respective pedal holders to simulate the walking gait path.

**SUMMARY OF THE INVENTION**

The present invention has been accomplished under the circumstances in view. It is the main object of the present invention to provide a gait training machine, which allows the user to know the movement of the ankle joint and the center-of-gravity of the body to adjust the healthy gait posture.

To achieve this and other objects of the present invention, a gait training machine comprising the features of claim 1 is provided. The gait training machine comprises a base, a driving module, a sensor module, a controller and a signal processing module. The driving module is disposed in the base. The driving module comprises a driving circuit, a motor and two moving platforms. The driving circuit controls the motor to move the moving platforms. The sensor module comprises a left pedal, a right pedal, a plurality of pressure sensors and a position sensor. The left pedal and the right pedal are respectively pivotally connected to the moving platforms. The pressure sensors are respectively mounted on the left pedal and the right pedal. The position sensor is disposed in the base. The controller has the function of storing, processing and outputting data, and is electrically connected to the driving circuit, each pressure sensor and the position sensor. The signal processing module is disposed in the controller, comprising a gait detecting unit and a gait judgment unit. The gait judgment unit stores a center-of-gravity calculation logic and a gait determining logic. The motor drives the moving platforms to move the left pedal and the right pedal in the opposite direction. In the turning point of the reciprocating movement of each moving platform, the motor is reversely rotated, and at this time, the driving circuit generates a steering signal. When the left pedal and the right pedal pass the position sensor with the moving platforms, the position sensor generates a passing signal. The gait detecting unit receives weight signals detected by the pressure sensors, the passing signal detected by the position sensor and the steering signal provided by the driving circuit. The gait judgment unit receives the weight signals, the passing signal and the steering signa from the gait detecting unit, and uses the center-of-gravity calculation logic to calculate the bearing weight of the left pedal and the right pedal and the position of the center-of-gravity on the left pedal and right right pedal, and also uses the gait determining logic to determine whether the bearing weight of the left pedal and the right pedal is correct and whether the position of the center-of-gravity is correct.

In this way, the user can know the movement of the ankle joint and the center-of-gravity of the body to adjust the health gait posture.

It is worth mentioning that the gait determining unit calculates the number of passes, the number of times of steering, the number of times that meet a first condition which indicates that the position of the center-of-gravity of said left pedal and said right pedal is correct, and the number of times that meet a second condition which indicates that the user's left foot and the right foot's ankle movement are correct. When the gait determining unit receives the passing signal, the number of passes is increased by one, and the gait determination logic is used to determine whether the bearing weight of the left pedal and the right pedal is correct. If correct, the number of times that meet said first condition is increased by one. If not, the number of times that meet said first condition is unchanged. When the gait judgment unit receives the steering signal, the number of times of steering is increased by one, and the gait determining logic is also used to determine whether the user's left foot and the right foot's ankle movement are correct. If correct, the number of times that meet said second condition is increased by one. If not, the number of times that meet said second condition remains unchanged.

Preferably, the gait training machine further comprises an evaluation module disposed at the controller. The evaluation module comprises a guiding unit. After the guiding unit receives the steering signal generated by the driving circuit, the guiding unit transmits a first guiding indicator to guide the user to move the center-of-gravity to one of the feet. After the guiding unit receives the steering signal again, the guiding unit transmits a second guiding indicator to guide the user to move the center-of-gravity to the other foot.

Preferably, the evaluation module further comprises a level-of-involvement evaluation unit that calculates an ankle joint correct rate and a center-of-gravity correct rate, the ankle joint correct rate = the number of times that meet said second condition /the number of times of steering * 100%, the center-of-gravity correct rate = the number of times that meet said first condition /the number of passes * 100%. This allows the user to evaluate the correct rate of gait posture.

The use of the gait training machine may comprise the steps of:

A) preparation stage: where the user puts the both feet on the left pedal and the right pedal respectively, and the touch screen is for the user to set a speed level, a step distance, and a training time;
B) gait training machine action stage: where the controller converts the speed level, the step length and the training time into a driving data, and outputs the driving data to the driving circuit of the driving module for the driving circuit to control the rotation of the motor, thereby controlling the movement speed, the movement time and the range of motion of each of the moving platforms to drive the left pedal and the right pedal; the range of motion of each moving platform is limited to the step length; at the reciprocating turning point of each moving platform, the motor produces a reverse rotation, at this time, the driving circuit generates a steering signal; when the left pedal and the right pedal move with the respective moving platforms to pass the position sensor, the position sensor generates a passing signal;
C) signal detection and judgment: where the gait detecting unit receives a weight signal from each pressure sensor, the passing signal measured by the position sensor and the steering signal from the driving circuit, the gait judgment unit receives each weight signal from the gait detecting unit, the passing signal and the steering signal and uses the center-of-gravity calculation logic to calculate the bearing weight of the left pedal and the right pedal and the center-of-gravity position on the left pedal and the right pedal and also uses the gait determining logic to determine whether the bearing weight of the eft pedal and the right pedal is correct and whether the center-of-gravity position is correct; the gait judgment unit calculates the number of passes, the number of times of steering, the number of times that meet a first condition which indicates that the position of the center-of-gravity of said left pedal and said right pedal is correct and the number of times that meet a second condition which indicates that the user's left foot and the right foot's ankle movement are correct; when the gait judgment unit receives the passing signal, the number of passes is increased by one, and the gait determining logic is used to determine whether the bearing weight of the left pedal and the right pedal is correct, if correct, the number of times that meet said first condition is increased by one, and if not, the number of times that meet said first condition is unchanged; when the gait judgment unit receives the steering signal, the number of times of steering is increased by one, and the gait determining logic is used to determine whether the user's left foot and the right foot's ankle movement are correct, if all are correct, then the number of times that meet said second condition is increased by one, and if not, the number of times that meet said second condition is unchanged;

D) guiding display: after receiving the steering signal from the driving circuit in the process that the left pedal moves forward and the right pedal moves backward, the guiding unit transmits a left guiding signal to the touch screen, at this time, the touch screen displays a left arrow for guiding the user to move the center-of-gravity to the left foot, then, after receiving the steering signal again, the guiding unit transmits a right guiding signal to said touch screen, at this time, said touch screen displays a right arrow for guiding the user to move the center-of-gravity to the right foot; in addition, the guiding unit also receives from the gait judgment unit the result of determining whether the user's left and right ankle movements are correct, and the touch screen displays a left-shoe icon, a right-shoe icon, if the left ankle joint moves correctly, a left ankle joint display signal is sent to the touch screen, and the left-shoe icon is colored or illuminated after receiving the left ankle joint display signal for the user to understand that the left ankle joint moves correctly, if the right ankle joint moves correctly, a right ankle joint display signal is sent to the touch screen, and the right-shoe icon is colored or illuminated after receiving the right ankle joint display signal for the user to understand that the right ankle joint moves correctly; and

E) repeated action: where steps B)-D) are repeated till said training time is ended.

In this way, the user can know and adjust the movement of the ankle joint and the center-of-gravity of the body to adjust the health gait posture.

It is worth mentioning that the valuation module further comprises a level-of-involvement evaluation unit, the method further comprises step F) after step E). In step F) result display: the level-of-involvement evaluation unit calculates an ankle joint correct rate and a center-of-gravity correct rate within the training time, the ankle joint correct rate = the number of times that meet said second condition /the number of times of steering * 100%, the center-of-gravity correct rate = the number of times that meet said first condition/the number of passes * 100%. The touch screen is able to be set to display the number of times that meet said second condition, the number of times of steering, the ankle joint correct rate, the number of times that meet said first condition, the number of passes, or the center-of-gravity correct rate.

It is worth mentioning that the determining logic is introduced as follows:

the gait judgment unit pre-stores a first threshold and a second threshold;

with respect to the center-of-gravity position of the user on the left pedal, the moving direction of the left pedal and the right pedal is the front-rear direction, and when the user stands on the left pedal and the right pedal, the side the user faces is the front side,

Total LValue: left pedal bearing weight;

Total RValue: right pedal bearing weight;

Total Value: the user's weight;

$$\text{Total Value} = \text{Total LValue} + \text{Total RValue;}$$

when the left pedal moves backward and the passing signal is received, Total LValue/Total Value=first threshold, it is determined that the user's body center-of-gravity is on the left foot, and the bearing weight of the left pedal is correct, if not, an error of the bearing weight of said left pedal is determined;

when the right pedal moves backward and the passing signal is received, Total RValue/Total Value=first threshold, it is determined that the user's body center-of-gravity is on the right foot, and the bearing weight of the right pedal is correct, if not, an error of the bearing weight of the right pedal is determined;

define the forward moving direction of the left pedal and the right pedal as the X direction and the outward direction perpendicular to the X direction as the Y direction;

when |LX Position| / (LX_Proportion/2) $\geqq$ the second threshold, it indicates that the left ankle joint movement is correct, if not,it indicates that the left ankle joint movement is wrong;

LX_Position: the coordinate value of the center-of-gravity of the X direction on said left pedal;

LX_Proportion: the length of the left pedal along the X direction with the geometric center of the left pedal as the origin;

when |RX Position| / (RX_Proportion/ 2) $\geqq$ the second threshold, it indicates that the right ankle joint movement is correct, if not,it indicates that the right ankle joint movement is wrong;

RX Position: the coordinate value of the center-of-gravity of the X direction on the right pedal;

RX_Proportion: the length of the right pedal along the X direction with the geometric center of the right pedal as the origin.

In addition, it must be stated that the number of the pressure sensors is eight, which are respectively arranged in the four corners of the left pedal and the right pedal, and the pressure sensors on the left rear side, left front side, right front side and right rear side of the left pedal are defined as sensor A, sensor B, sensor C and sensor D respectively, and the pressure sensors on the right rear side, right front side, left front side and left rear side of the right pedal are defined as sensor E, sensor F, sensor G and sensor H respectively. When the user stands on the left pedal and the right pedal, the weight is applied to the left pedal and the right pedal;

$$\text{Total LValue} = \text{LValue1} + \text{LValue2} + \text{LValue3} + \text{LValue4};$$

Total LValue: left pedal bearing weight;
LValue1: the weight signal detected by said sensor A;
LValue2: the weight signal detected by said sensor B;
LValue3: the weight signal detected by said sensor C;
LValue4: the weight signal detected by said sensor D;

$$\text{Total RValue} = \text{RValue1} + \text{RValue2} + \text{RValue3} + \text{RValue4};$$

Total RValue: right pedal bearing weight;
RValue1: the weight signal detected by said sensor E;
RValue2: the weight signal detected by said sensor F;
RValue3: the weight signal detected by said sensor G;
RValue4: the weight signal detected by said sensor H;

$$\text{Total Value} = \text{Total LValue} + \text{Total RValue};$$

Total Value: the user's weight.
then the center-of-gravity position on the left pedal is obtained by the following relationship:

$$\text{LX\_Gravity} = ((\text{LValue3} + \text{LValue2}) * \text{LX\_Proportion})/\text{Total LValue};$$

$$\text{LY\_Gravity} = ((\text{LValue2} + \text{LValue1}) * \text{LY\_Proportion})/\text{Total LValue};$$

LValue1: the weight signal detected by said sensor A;
LValue2: the weight signal detected by said sensor B;
LValue3: the weight signal detected by said sensor C;
LX_Proportion = the length of the left pedal along the X direction with the geometric center of the left pedal as the origin;
LY_Proportion = the length of the left pedal along the Y direction with the geometric center of the left pedal as the origin;
thus, the center-of-gravity coordinates on the left pedal (LX_Position, LY_Position) is obtained by the following relationship:

$$\text{LX\_Position} = \text{LX\_Gravity} - (\text{LX\_Proportion}/2);$$

$$\text{LY\_Position} = \text{LY\_Gravity} - (\text{LY\_Proportion}/2);$$

the center-of-gravity position on said right pedal is obtained by the following relationship:

$$\text{RX\_Gravity} = ((\text{RValue3} + \text{RValue2}) * \text{RX\_Proportion})/\text{Total RValue};$$

$$RY\_Gravity = ((RValue2 + RValue1) * LY\_Proportion)/Total\ RValue;$$

RValue1: the weight signal detected by said sensor E;
RValue2: the weight signal detected by said sensor F;
RValue3: the weight signal detected by said sensor G;
RX_Proportion: the length of the right pedal along the X direction with the geometric center of the right pedal as the origin;
RY_Proportion: the length of the right pedal along the Y direction with the geometric center of the right pedal as the origin;
the center-of-gravity coordinates on the right pedal (RX_Position, RY_Position) is obtained by the following relationship:

$$RX\_Position = RX\_Gravity - (RX\_Proportion/2);$$

$$RY\_Position = RY\_Gravity - (RY\_Proportion/2).$$

It should be noted that the first threshold is 0.8 and the second threshold is 0.8.
To further guide the user's gait action, in the step D) guiding display, the left-shoe icon comprises a left-heel portion and a left-sole portion, and the right-shoe icon comprises a right-heel portion and a right-sole portion. When the guiding unit receives the steering signal during the process that the left pedal moves forward and the right pedal moves backward, the guiding unit transmits a first display signal to the touch screen, at this time, said left-heel portion is colored or illuminated to guide the user to put the center-of-gravity of the left foot on the left heel and also said right-sole portion is colored or illuminated to guide the user to put the center-of-gravity of the right foot on the right sole; then, the left pedal moves backward and the right pedal moves forward; when the guiding unit receives the passing signal, the guiding unit transmits a second display signal to the touch screen, at this time, the left-sole portion is colored or illuminated to guide the user to put the center-of-gravity of the left foot on the left sole and also the right-heel portion is colored or illuminated to guide the user to put the center-of-gravity of the right foot on the right heel.
Other advantages and features of the present invention will be fully understood by reference to the following specification in conjunction with the accompanying drawings, in which like reference signs denote like components of structure.

**BRIEF DESCRIPTION OF THE DRAWINGS**

FIG. 1 is an oblique top elevational view of a gait training machine in accordance with the present invention.
FIG. 2 is a rear elevation of a part of the gait training machine in accordance with the present invention.
FIG. 3 is a partial elevational view of the gait training machine in accordance with the present invention, illustrating the structure of the left pedal.
FIG. 4 is a partial elevational view of the gait training machine in accordance with the present invention, illustrating the structure of the right pedal.
FIG. 5 is a system block diagram of the gait training machine in accordance with the present invention, illustrating the arrangement of the controller, the driving module, the pressure sensor and the position sensor.

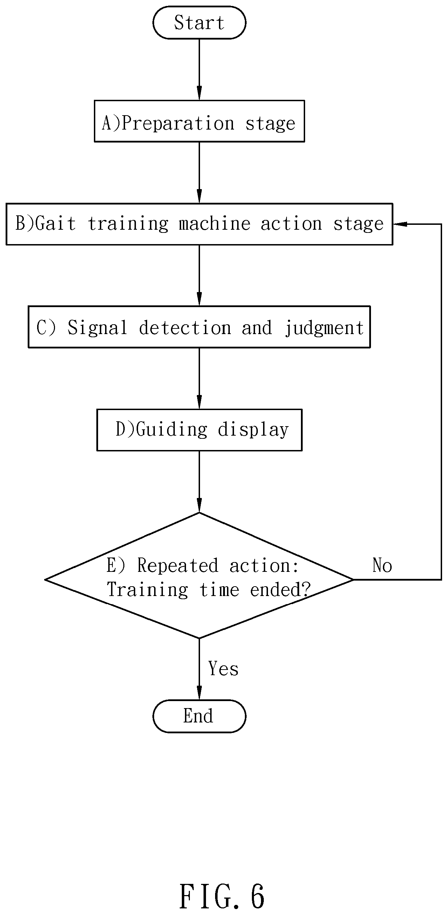

FIG. 6 is a flow chart of the use of a gait training machine of the present invention, showing steps A)-E).
FIG. 7 is a schematic drawing of the gait training machine using method in accordance with the present invention, showing the distribution of the center-of-gravity coordinate position of the left pedal.
FIG. 8 is a schematic drawing of the gait training machine using method in accordance with the present invention, showing the distribution of the center-of-gravity coordinate position of the right pedal.
FIG. 9 is a schematic drawing of the use of the gait training machine of the present invention, showing a left arrow displayed on the touch screen to guide the user to move the center-of-gravity of the body to the left foot.
FIG. 10 is a schematic drawing of the use of the gait training machine of the present invention, showing a right arrow displayed on the touch screen to guide the user to move the center-of-gravity of the body to the right foot.
FIG. 11 is a schematic drawing of the use of the gait training machine of the present invention, showing a left-shoe icon and a right-shoe icon displayed on the touch screen.
FIG. 12 is a schematic drawing of the use of the gait training machine of the present invention, showing a left-heel portion, a left-sole portion, a right-heel portion and a right-sole portion displayed on the touch screen.

FIG. 13 is a flow chart of the use of the gait training machine of the present invention, showing steps A)-F).

## DETAILED DESCRIPTION OF THE INVENTION

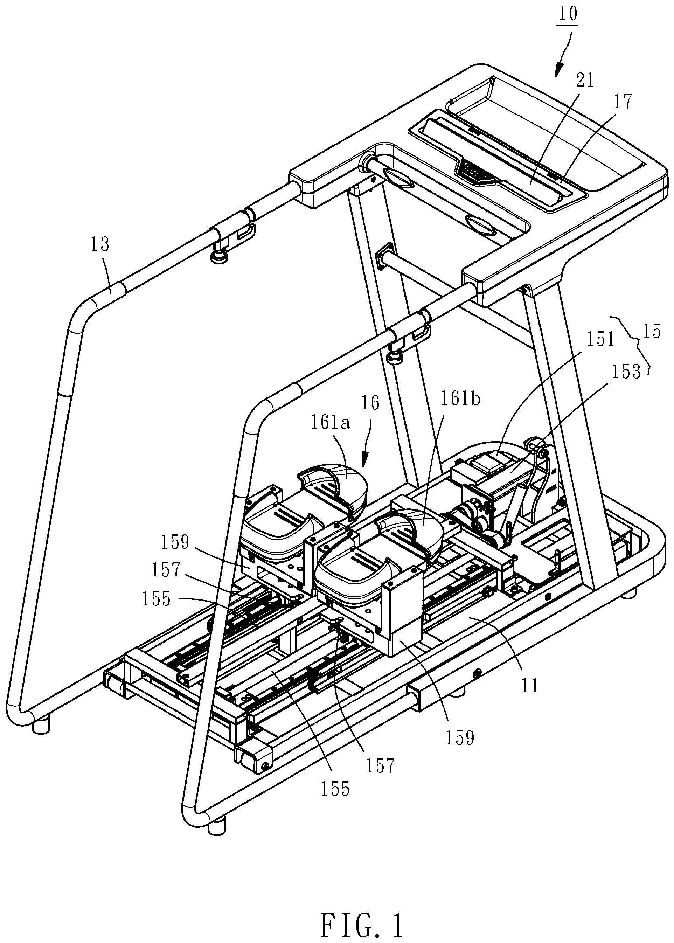

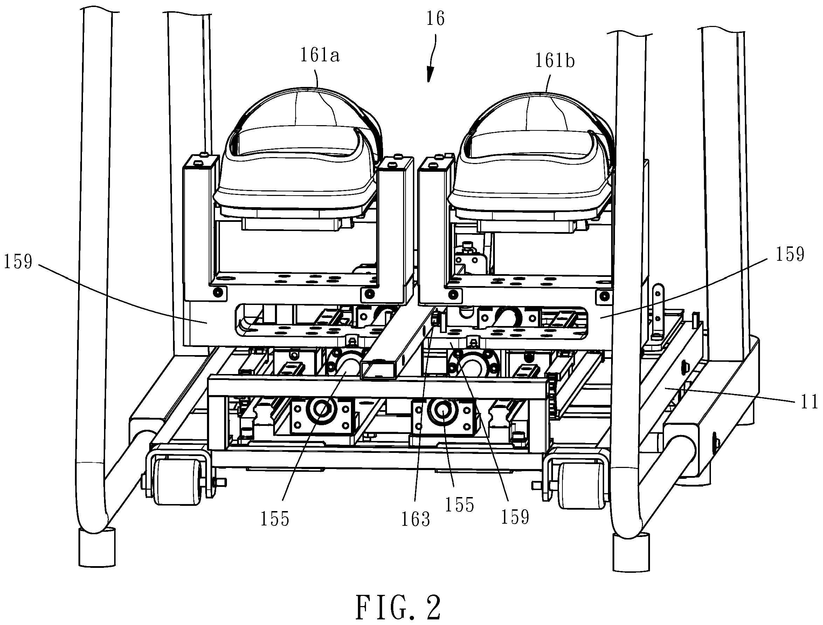

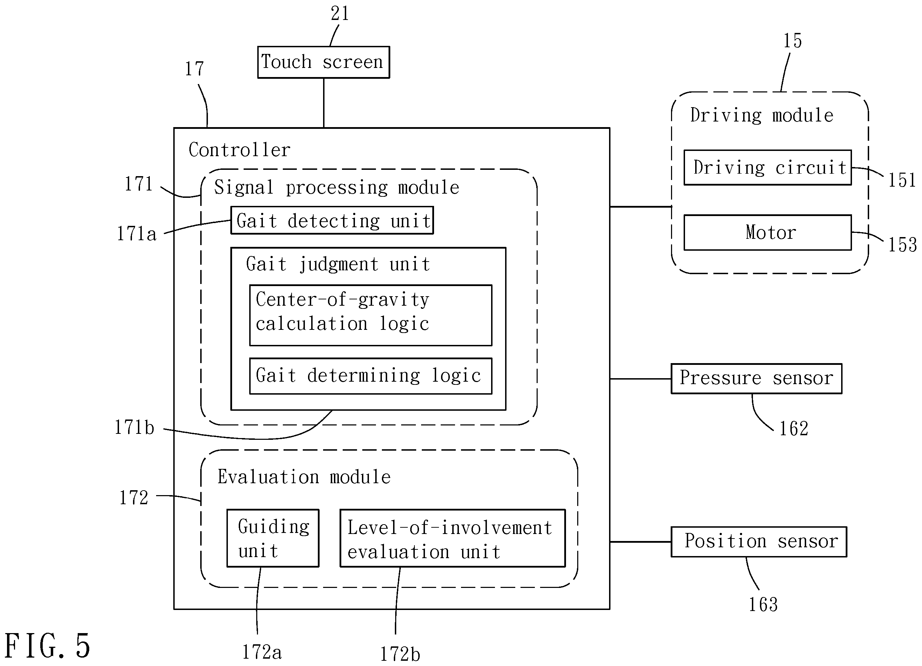

Referring to FIGS. 1-5, the present invention provides a gait training machine **10**. The gait training machine **10** includes a base **11**, an armrest **13**, a driving module **15**, a sensing module **16**, a controller **17**, a signal processing module **171** and an evaluation module **172**. The base **11** is set on the ground.

The armrest **13** is mounted on the base **11** for the user to rest the arms when performing gait training. It should be noted that the armrest **13** is a component that assists the user and can be installed or removed.

The driving module **15** is disposed inside the base **11**. The driving module **15** comprises a driving circuit **151** and a motor **153**. The driving circuit **151** is used to control the motor **153**.

The motor **153** drives two screw rods **155**. Two screw nuts **157** are respectively threaded onto the two screw rods **155**. Two moving platforms **159** are respectively pivotally connected to the two screw nuts **157** and moved with the screw nuts **157** along the respective screw rods **155**. The sensor module **16** comprises a left pedal **161a**, a right pedal **161b**, a plurality of pressure sensors **162**, and a position sensor **163**.

The left pedal **161a** and the right pedal **161b** are respectively pivotally connected to the respective moving platforms **159**. When the user's feet are respectively stepped on the left pedal **161a** and the right pedal **161b**, the moving platforms **159** move interactively to drive the user's gait movement, causing the left pedal **161a** and the right pedal **161b** to swing as the user's ankle joints rotate. The pressure sensors **162** are respectively disposed on the left pedal **161a** and the right pedal **161b** and used to measure the weight applied by the user's feet to generate a weight signal. It is worth mentioning that each pressure sensor **162** can be replaced by a load cell or a gyroscope.

The position sensor **163** is disposed in the base **11** on one side of the screw rods **155**. When the moving platform **159** passes the position sensor **163**, the position sensor **163** generates a passing signal. It is worth mentioning that in this embodiment, the position sensor **163** is a proximity switch, but any sensor capable of detecting the passage of an object can be used here, such as a photoelectric sensor, an electromagnetic sensor, or a capacitive sensor, etc.

The controller **17** has the function of storing, processing and outputting data.

The controller **17** is electrically connected to the driving circuit **151**, each pressure sensor **162** and the position sensor **163**.

The signal processing module **171** is disposed in the controller **17**, comprising a gait detecting unit **171a** and a gait judgment unit **171b**. The gait judgment unit **171b** stores a center-of-gravity calculation logic and a gait determining logic.

Wherein, the motor **153** drives the moving platforms **159** to move the left pedal **161a** and the right pedal **161b** in the opposite direction. In the turning point of the reciprocating movement of each moving platform **159**, the motor **153** is reversely rotated. At this time, the driving circuit **151** generates a steering signal. When the left pedal **161a** and the right pedal **161b** pass the position sensor **163** with the moving platforms **159**, the position sensor **163** generates a passing signal.

Wherein, the gait detecting unit **171a** receives the weight signals detected by the pressure sensors **162**, the passing signal detected by the position sensor **163** and the steering signal provided by the driving circuit **151**. The gait judgment unit **171b** receives the weight signals, the passing signal and the steering signa from the gait detecting unit **171a**, and uses the center-of-gravity calculation logic to calculate the bearing weight of the left pedal **161a** and the right pedal **161b** and the position of the center-of-gravity on the left pedal **161a** and the right pedal **161b**, and also uses the gait determining logic to determine whether the bearing weight of the left pedal **161a** and the right pedal **161b** is correct and whether the position of the center-of-gravity is correct.

With the above-mentioned gait training machine, the user can know the movement of the ankle joint and the center-of-gravity of the body to adjust the healthy gait posture.

It is worth mentioning that the gait judgment unit **171b** can calculate the number of passes, the number of times of steering, the number of times that meet a first condition which indicates that the position of the center-of-gravity of said left pedal and said right pedal is correct, and the number of times that meet a second condition which indicates that the user's left foot and the right foot's ankle movement are correct. When the gait judgment unit **171b** receives the passing signal, the number of passes is increased by one. The gait determination logic is used to determine whether the bearing weight of the left pedal **161a** and the right pedal **161b** is correct. If correct, the number of times that meet said first condition is increased by one. If not, the number of times that meet said first condition is unchanged. When the gait judgment unit **171b** receives the steering signal, the number of times of steering is increased by one. Further, the gait determining logic is also used to determine whether the user's left foot and the right foot's ankle movement are correct. If correct, the number of times that meet said second condition is increased by one. If not, the number of times that meet said second condition remains unchanged.

In addition, this embodiment also selects to include an evaluation module **172**. The evaluation module **172** is disposed at the controller **17,** comprising a guiding unit **172a.** After the guiding unit **172a** receives the steering signal generated by the driving circuit **151,** the guiding unit **172a** transmits a first guiding indicator to guide the user to move the center-of-gravity to one of the feet. After the guiding unit **172a** receives the steering signal again, the guiding unit **172a** transmits a second guiding indicator to guide the user to move the center-of-gravity to the other foot.

In this embodiment, the evaluation module **172** further comprises a level-of-involvement evaluation unit **172b** that calculates an ankle joint correct rate and a center-of-gravity correct rate. The ankle joint correct rate = the number of times that meet said second condition /the number of times of steering * 100%. The center-of-gravity correct rate = the number of times that meet said first condition/the number of passes * 100%. It also allows the user to know the movement of the ankle joint and the movement of the center-of-gravity of the body.

The above is the introduction of the gait training machine **10** provided by the present invention. It should be noted that when the gait training machine provided by the first embodiment of the present invention is used, the controller 17 may be electrically connected to a touch screen **21.** As shown in FIG. 1, the touch screen **21** is used for inputting data by the user and displaying information.

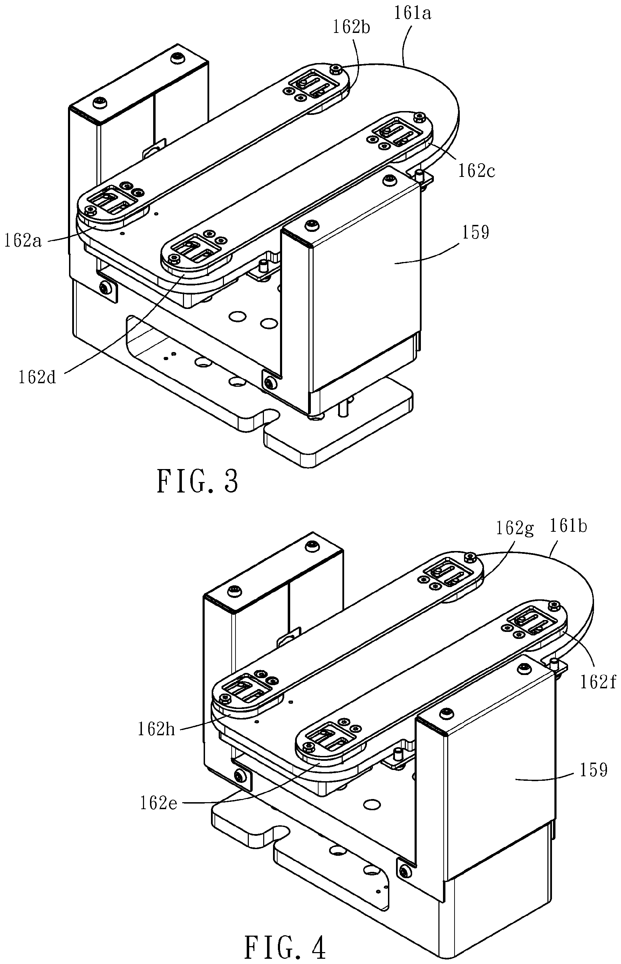

As shown in FIGS. 3 and 4, the number of the pressure sensors **162** is eight, which are respectively disposed at the four corners of the left pedal **161a** and the right pedal **161b.** The pressure sensors **162** on the left rear side, left front side, right front side and right rear side of the left pedal **161a** are sensor **A** (**162a**), sensor **B** (**162b**), sensor **C** (**162c**) and sensor **D** (**162d**) respectively. The pressure sensors **162** on the right rear side, right front side, left front side and left rear side of the right pedal **161b** are sensor **E** (**162e**), sensor **F** (**162f**), sensor **G** (**162g**), sensor **H** (**162h**) respectively.

Using the gait training machine **10** may include the following steps:

A) Preparation stage: The user puts the both feet on the left pedal **161a** and the right pedal **161b** respectively. The touch screen **21** is for the user to set a speed level, a step distance, and a training time.

The controller **17** displays a plurality of speed levels on the touch screen **21** for the user to select one of the speed levels.

Each speed level indicates the moving speed of the moving platform **159,** from fast to slow or from slow to fast. For example, the moving platform **159's** moving speed is divided into 4 levels. As a result of slow to fast, the moving speed of the moving platform 159 at the fourth level is faster than the third level faster than the second level faster than the second level. It should be noted that when the moving platform **159** reciprocates, its speed distribution can be divided into an acceleration section, a constant speed section and a deceleration section. Here, the speed level refers to the moving speed grading of the moving platform at the constant speed section.

The controller **17** also displays a step length field on the touch screen **21** for the user to input a step length. The step length in the gait training machine **10** reflects the distance of the corresponding position on the left pedal **161a** and the right pedal **161b,** for example, the distance between the rear end point of the left pedal **161a** and the rear end point of the right pedal **161b** is 80 cm, that is, input 80 cm.

The controller **17** also displays a gait training time field on the touch screen **21** for the user to input a training time data, for example, 30 minutes, 40 minutes.

B) Gait training machine action stage:

The controller **17** converts the speed level, the step length and the training time into a driving data, and outputs the driving data to the driving circuit **151** of the driving module **15,** for the driving circuit **151** to control the rotation of the motor **153,** thereby controlling the movement speed, the movement time and the range of motion of each of the moving platforms **159** to drive the left pedal **161a** and the right pedal **161b.** The range of motion of each moving platform **159** is limited to the step length. At the reciprocating turning point of each moving platform **159,** the motor **153** produces a reverse rotation. At this time, the driving circuit **151** generates a steering signal. When the left pedal **161a** and the right pedal **161b** move with the respective moving platforms **159** to pass the position sensor **163,** the position sensor **163** generates a passing signal.

Here, after the left pedal **161a** or the right pedal **161b** is located at the turning point in the moving direction, the next moving direction will be switched, for example, the left pedal **161a** (or the right pedal **161b**) moves forward to the end of the stroke, and then moves backward; the left pedal **161a** (or the right pedal **161b**) moves backward to the end of the stroke, and then moves forward.

C) Signal detection and judgment:

The gait detecting unit **171a** receives a weight signal from each pressure sensor **162,** the passing signal measured by the position sensor **163** and the steering signal from the driving circuit **151.** The gait judgment unit **171b** receives each weight signal from the gait detecting unit **171a,** the passing signal and the steering signal, and uses the center-of-gravity calculation logic to calculate the bearing weight of the left pedal **161a** and the right pedal **161b** and the center-of-gravity position on the left pedal **161a** and the right pedal **161b,** and also uses the gait determining logic to determine whether the bearing weight of theleft pedal **161a** and the right pedal **161b** is correct and whether the center-of-gravity position is correct. The gait judgment unit **171b** calculates the number of passes, the number of times of steering, the number of times that meet a first condition which indicates that the position of the center-of-gravity of said left pedal and said right pedal is correct, the number of times that meet a second condition which indicates that the user's left foot and the right foot's ankle movement are correct. When the gait judgment unit **171b** receives the passing signal, the number of passes is increased by one, and the gait determining logic is used to determine whether the bearing weight of the left pedal **161a** and the right pedal **161b** is correct. If correct, the number of times that meet said first condition is increased by one, and if not, the number of times that meet said first condition is unchanged. When the gait judgment unit **171b** receives the steering signal, the number of times of steering is increased by one, and the gait determining logic is used to determine whether the user's left foot and the right foot's ankle movement are correct. If all are correct, then the number of times that meet said second condition is increased by one, and if not, the number of times that meet said second condition is unchanged.

The center-of-gravity calculation logic is described below.

When the user stands on the pedals, the weight is applied to the left pedal **161a** and the right pedal **161b.**

$$\text{Total LValue} = \text{LValue1} + \text{LValue2} + \text{LValue3} + \text{LValue4};$$

Total LValue: left pedal bearing weight;
LValue1-4: weight signals of sensors A-D;

$$\text{Total RValue} = \text{RValue1} + \text{RValue2} + \text{RValue3} + \text{RValue4};$$

Total RValue: right pedal bearing weight;
RValue1-4: the weight signals of sensors E-H;

$$\text{Total Value} = \text{Total LValue} + \text{Total RValue};$$

Total Value: the user's weight.

Then, calculate the center-of-gravity position.

With respect to the center-of-gravity position of the user on the left pedal 161a, the moving direction of the left pedal **161a** and the right pedal **161b** is the front-rear direction. When the user stands on the left pedal **161a** and the right pedal **161b,** the front side faces the X direction. The outward direction perpendicular to the X direction is the Y direction. Under this circumstance, the center-of-gravity position is calculated by the following relationship:

$$\text{LX\_Gravity} = ((\text{LValue3} + \text{LValue2}) * \text{LX\_Proportion})/\text{Total LValue};$$

$$\text{LY\_Gravity} = ((\text{LValue2} + \text{LValue1}) * \text{LY\_Proportion})/\text{Total LValue};$$

LValue1-3: Weight signals of sensors A-C;
LX_Proportion = the length of the left pedal along the X direction with the geometric center of the left pedal **161a** as the origin;
LY_Proportion = the length of the left pedal along the Y direction with the geometric center of the left pedal **161a** as the origin;
Thus, the center-of-gravity coordinates is on the left pedal **161a:**

$$\text{LX\_Position} = \text{LX\_Gravity} - (\text{LX\_Proportion}/2);$$

$$LY\_Position = LY\_Gravity - (LY\_Proportion/2);$$

(LX_Position, LY_Position) = Center-of-gravity coordinates on the left pedal **161a.**

The center-of-gravity position on the right pedal **161b**:

$$RX\_Gravity = ((RValue3 + RValue2) * RX\_Proportion)/Total\ RValue;$$

$$RY\_Gravity = ((RValue2 + RValue1) * LY\_Proportion)/Total\ RValue;$$

RValue1-3: Weight signals of sensors E-G
RX_Proportion = the length of the right pedal along the X direction with the geometric center of the right pedal **161b** as the origin;
RY_Proportion = the length of the right pedal along the Y direction with the geometric center of the right pedal **161b** as the origin.

Thus, the center-of-gravity coordinates on the right pedal **161b**:

$$RX\_Position = RX\_Gravity - (RX\_Proportion/2);$$

$$RY\_Position = RY\_Gravity - (RY\_Proportion/2);$$

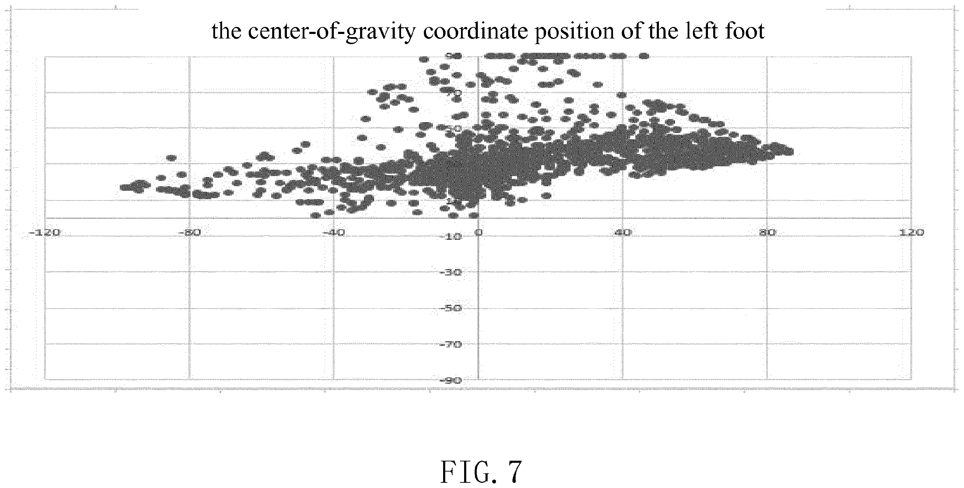

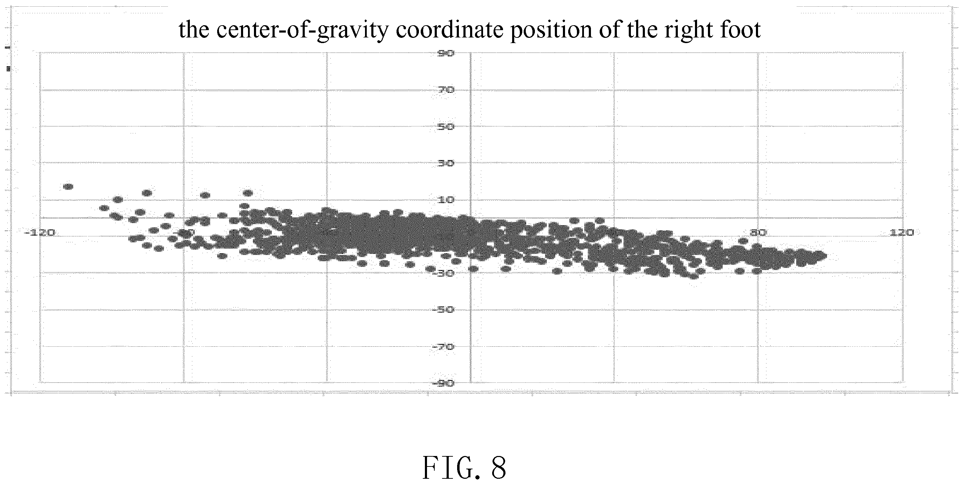

(RX_Position, RY_Position) = the center-of-gravity coordinates on the right pedal **161b.**
As shown in FIG. 7, the gravity center coordinate position distribution diagram on the left pedal **161a** is exemplified, the X direction coordinate indicates the length of the left pedal **161a,** and the Y coordinate indicates the width of the left pedal **161a.** As shown in FIG. 8, the gravity center coordinate position distribution diagram on the right pedal **161b** is exemplified, the X direction coordinate indicates the length of the right pedal **161b,** and the Y coordinate indicates the width of the left pedal **161a.** The value of each point in FIGS. 7 and 8 is the center-of-gravity position of the left pedal **161a** or right pedal **161b** in the training time calculated by the center-of-gravity calculation logic by a preset sampling frequency. For example, when the sampling frequency is 0.2 (Hz), it means that the pressure sensors **162** on the left pedal **161a** and the right pedal **161b** measure the weight signal once per every 5 seconds. The sampling frequency can also be set to other values, not limited to this.
The gait determining logic is introduced as follows:
The gait judgment unit **171b** pre-stores a first threshold and a second threshold.
When the left pedal **161a** moves backward and the passing signal is received, Total LValue/Total Value=first threshold, it is determined that the user's body center-of-gravity is on the left foot, and the bearing weight of the left pedal **161a** is correct; if not, an error of the bearing weight of the left pedal **161a** is determined.
When the right pedal **161a** moves backward and the passing signal is received, Total RValue/Total Value=first threshold, it is determined that the user's body center-of-gravity is on the right foot, and the bearing weight of the right pedal **161a** is correct; if not, an error of the bearing weight of the right pedal **161a** is determined.
It is worth mentioning that the first threshold is 0.8, but a value of 0.5 or more may be selected according to requirements; if the first threshold is lower than 0.5, the center-of-gravity of the body is on the other side, and there is no benefit of determining the center-of-gravity of the body; if the first threshold is higher than 1, the unilateral body weight is higher than the whole body weight, which is a wrong situation.
In addition, the gait judgment unit **171b** also pre-stores the second threshold.

|LX Position| / (LX_Proportion/2)$\geq$ said second threshold, it indicates that the left ankle joint is moving correctly; if not, it indicates that the left ankle joint movement is wrong;
|RX Position|/ (RX_Proportion/2)$\geq$ said second threshold, it indicates that the right ankle joint is moving correctly; if not, it indicates that the right ankle joint movement is wrong.

The second threshold is 0.8, but a value of 0.5 or more may also be selected according to requirements. Since |LX Position| represents the distance of the center-of-gravity position on the left pedal **161a** from the origin of the left pedal **161a** in the X direction, |RX Position I represents the distance of the center-of-gravity position on the right pedal **161b** from the origin of the right pedal 161b in the X direction, so the greater the value of |LX Position| and |RX Position|, the greater the angle of ankle joint movement and the greater the degree of ankle joint movement. Therefore, if the second threshold is lower than 0.5, the movement angle of the ankle joint is insufficient, and there is no training benefit; if the second threshold is higher than 1, the center-of-gravity coordinates is outside the left and the right pedal, which is a wrong situation.

D) Guiding display:

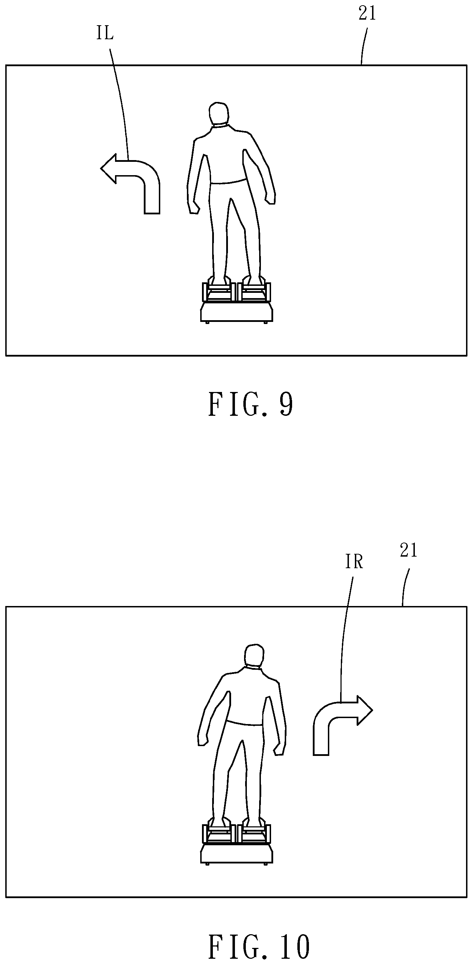

As shown in FIGS. 9 and 10, after receiving the steering signal from the driving circuit **151** in the process that the left pedal **161a** moves forward and the right pedal **161b** moves backward, the guiding unit **172a** transmits a left guiding signal to the touch screen **21**. At this time, the touch screen **21** displays a left arrow **IL** for guiding the user to move the center-of-gravity to the left foot. Then, after receiving the steering signal again, the guiding unit **172a** transmits a right guiding signal to the touch screen **21.** At this time, the touch screen **21** displays a right arrow **IR** for guiding the user to move the center-of-gravity to the right foot. Such displays are cycled to guide the user to change the position of the center-of-gravity in the gait.

After receiving the steering signal from the driving circuit **151** in the process that the left pedal **161a** moves backward and the right pedal **161b** moves forward, the guiding unit **172a** transmits the right guiding signal to the touch screen **21**. At this time, the touch screen **21** displays the right arrow **IR** for guiding the user to move the center-of-gravity to the right foot. Then, after receiving the steering signal again, the guiding unit **172a** transmits the left guiding signal to the touch screen **21**. At this time, the touch screen **21** displays the left arrow **IL** for guiding the user to move the center-of-gravity to the left foot. Such displays are cycled to guide the user to change the position of the center-of-gravity in the gait.

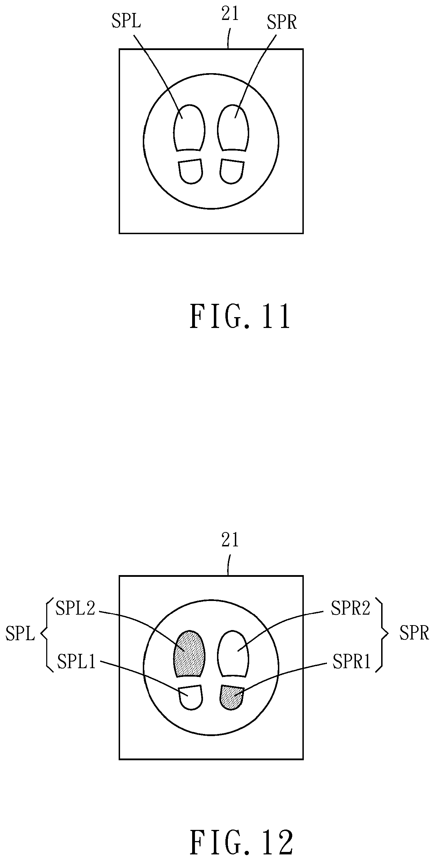

In addition, as shown in FIG. 11, the guiding unit **172a** also receives a result from the gait judgment unit **171b** to determine whether the ankle joint movement of the left and right feet of the user is correct.

In addition, as shown in FIG. 11, the guiding unit **172a** also receives from the gait judgment unit **171b** the result of determining whether the user's left and right ankle movements are correct. The touch screen **21** displays a left-shoe icon **SPL**, a right-shoe icon **SPR.** If the left ankle joint moves correctly, a left ankle joint display signal is sent to the touch screen **21,** and the left-shoe icon **SPL** is colored or illuminated after receiving the left ankle joint display signal for the user to understand that the left ankle joint moves correctly. If the right ankle joint moves correctly, a right ankle joint display signal is sent to the touch screen **21,** and the right-shoe icon **SPR** is colored or illuminated after receiving the right ankle joint display signal for the user to understand that the right ankle joint moves correctly. The display cycle is repeated for the user to understand the correct movement of the left and right ankle joints.

It is worth mentioning that, as shown in FIG. 12, the left-shoe icon **SPL** displayed by the touch screen **21** can be further divided into a left-heel portion **SPL1** and a left-sole portion **SPL2,** and the right-shoe icon **SPR** is further divided into right-heel portion **SPR1** and right-sole portion **SPR2**.

After receiving the steering signal from the driving circuit **151** in the process that the left pedal **161a** moves forward and the right pedal **161b** moves backward, the guiding unit **172a** simultaneously transmits a first display signal to the touch screen **21**. After receiving the left heel display signal, the touch screen **21** colors or illuminates the left-heel portion **SPL1** to indicate the user to put the center-of-gravity of the left foot on the left heel. After receiving the right sole display signal, the touch screen **21** colors or illuminates the right-sole portion **SPR2** to indicate the user to put the center-of-gravity of the right foot on the right sole. Then, after receiving the passing signal of the position sensor **163,** a second display signal are simultaneously sent to the touch screen **21.**

After receiving the left sole display signal, the touch screen **21** colors or illuminates the left-sole portion **SPL2** to indicate the user to put the center-of-gravity of the left foot on the left sole. After receiving the right heel display signal, the touch screen **21** colors or illuminates the right-heel portion **SPR1** to indicate the user to put the center-of-gravity of the right foot on the right heel.

E) Repeated action:

Repeat steps B)-D) until the training time is up.

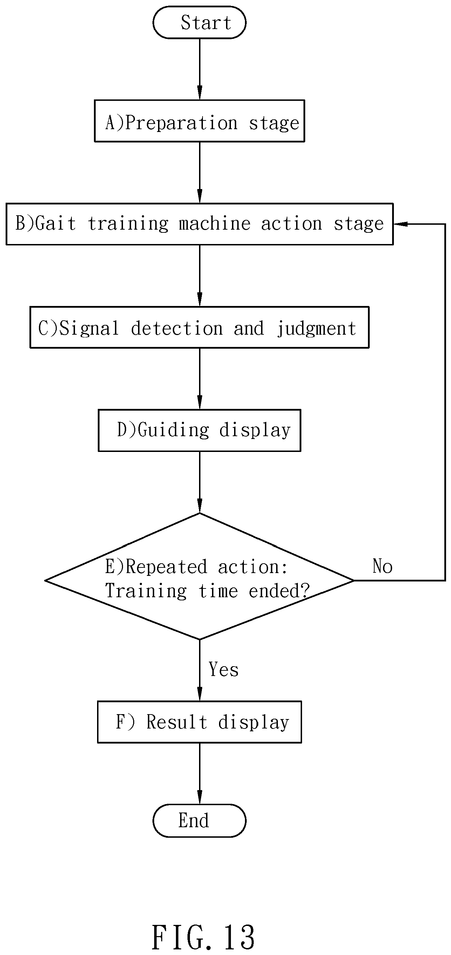

It is worth mentioning that, as shown in FIG. 13, step F) may be added after step E).

F) Result display:

The level-of-involvement evaluation unit **172b** calculates an ankle joint correct rate and a center-of-gravity correct rate within the training time, wherein the ankle joint correct rate = the number of times that meet said second condition /the number of times of steering * 100%; the center-of-gravity correct rate = the number of times that meet said first condition/the number of passes * 100%; the touch screen 21 can be set to display the number of times that meet said second condition, the number of times of steering, the ankle joint correct rate, the number of times that meet said first condition, the number of passes, or the center-of-gravity correct rate.

In this way, during the gait training, the user is informed of the ankle movement and the center-of-gravity of the body to adjust the healthy gait posture.

## Claims

1. A gait training machine (10), comprising a base (11), said gait training machine (10) further comprising:

   a driving module (15) disposed in said base (11), said driving module (15) comprising a driving circuit (151), a motor (153) and two moving platforms (159), said driving circuit (151) controlling said motor (153) to move said moving platforms (159);
   a sensor module (16) comprising a left pedal (161a), a right pedal (161b), a plurality of pressure sensors (162) or load cells or gyroscopes, and a position sensor (163), said left pedal (161a) and said right pedal (161b) being respectively pivotally connected to said moving platforms (159), said pressure sensors (162) or load cells or gyroscopes, being respectively mounted on said left pedal (161a) and said right pedal (161b), said position sensor (163) being disposed in said base (11);
   a controller (17) having the function of storing, processing and outputting data, said controller (17) being electrically connected to said driving circuit (151), each said pressure sensor (162) or load cell or gyroscope, and said position sensor (163);
   a signal processing module (171) disposed in said controller (17), said signal processing module (171) comprising a gait detecting unit (171a) and a gait judgment unit (171b), said gait judgment unit (171b) storing a center-of-gravity calculation logic and a gait determining logic;
   wherein, said motor (153) drives said moving platforms (159) to move said left pedal (161a) and said right pedal (161b) in the opposite direction; in the turning point of the reciprocating movement of each said moving platform (159), said motor (153) is reversely rotated, and at this time, said driving circuit (151) generates a steering signal; when said left pedal (161a) and said right pedal (161b) pass said position sensor (163), said position sensor (163) generates a passing signal;
   wherein, said gait detecting unit (171a) receives weight signals detected by said pressure sensors (162) or load cells or gyroscopes, said passing signal detected by said position sensor (163) and said steering signal provided by said driving circuit (151); said gait judgment unit (171b) receives said weight signals, said passing signal and said steering signal from said gait detecting unit (171a), and uses said center-of-gravity calculation logic to calculate the bearing weight of said left pedal (161a) and said right pedal (161b) and the position of the center-of-gravity on said left pedal (161a) and said right pedal (161b), and also uses said gait determining logic to determine whether the bearing weight of said left pedal (161a) and said right pedal (161b) is correct and whether the position of the center-of-gravity of said left pedal (161a) and said right pedal (161b) is correct.

2. The gait training machine (10) as claimed in claim 1, wherein said gait determining unit calculates the number of passes, the number of times of steering, the number of times that meet a first condition which indicates that the position of the center-of-gravity of said left pedal (161a) and said right pedal (161b) is correct, and the number of times that meet a second condition which indicates that the user's left foot and the right foot's ankle movement are correct; when said gait determining unit receives said passing signal, the number of passes is increased by one, and said gait determination logic is used to determine whether the bearing weight of said left pedal (161a) and said right pedal (161b) is correct, if correct, the number of times that meet said first condition is increased by one, if not, the number of times that meet said first condition is unchanged; when said gait judgment unit (171b) receives said steering signal, the number of times of steering is increased by one, and said gait determining logic is also used to determine whether the user's left foot and the right foot's ankle movement are correct, if correct, the number of times that meet said second condition is increased by one, if not, the number of times that meet said second condition remains unchanged.

3. The gait training machine (10) as claimed in claim 2, further comprising an evaluation module (172) disposed at said controller (17), said evaluation module (172) comprising a guiding unit (172a); after said guiding unit (172a) receives said steering signal generated by said driving circuit (151), said guiding unit (172a) transmits a first guiding indicator to guide the user to move the center-of-gravity to one of the feet; after said guiding unit (172a) receives said steering signal again, said guiding unit (172a) transmits a second guiding indicator to guide the user to move the center-of-gravity to the other foot.

4. The gait training machine (10) as claimed in claim 3, wherein said evaluation module (172) further comprises a level-of-involvement evaluation unit (172b) that calculates an ankle joint correct rate and a center-of-gravity correct rate, the ankle joint correct rate = the number of times that meet said second condition /the number of times of steering * 100%, the center-of-gravity correct rate = the number of times that meet said first condition /the number of passes * 100%.


**Patentansprüche**

1. Gangtrainingsmaschine (10), welche eine Basis (11) umfasst, worin die Gangtrainingsmaschine (10) ferner umfasst:

ein Antriebsmodul (15), das in der Basis (11) angeordnet ist, worin das Antriebsmodul (15) eine Antriebsschaltung (151), einen Motor (153) und zwei bewegliche Plattformen (159) umfasst, worin die Antriebsschaltung (151) den Motor (153) steuert, um die beweglichen Plattformen (159) zu bewegen;

ein Sensormodul (16), das ein linkes Pedal (161a), ein rechtes Pedal (161b), mehrere Drucksensoren (162) oder Lastmesszellen oder Gyroskopen und einen Positionssensor (163) umfasst, worin das linke Pedal (161a) und das rechte Pedal (161b) jeweils schwenkbar mit den sich bewegenden Plattformen (159) verbunden sind, worin die Drucksensoren (162) oder Lastmesszellen oder Gyroskope jeweils an dem linken Pedal (161a) und dem rechten Pedal (161b) angebracht sind, und der Positionssensor (163) in der Basis (11) angeordnet ist;

eine Steuerung (17) mit der Funktion des Speicherns, Verarbeitens und Ausgebens von Daten, worin die Steuerung (17) mit der Antriebsschaltung (151), jedem Drucksensor (162) oder jeder Kraftmesszelle oder jedem Gyroskop und dem Positionssensor (163) elektrisch verbunden ist;

ein Signalverarbeitungsmodul (171), das in der Steuerung (17) angeordnet ist, worin das Signalverarbeitungsmodul (171) eine Gangerkennungseinheit (171a) und eine Gangbeurteilungseinheit (171b) umfasst, worin die Gangbeurteilungseinheit (171b) eine Schwerpunktsberechnungslogik und eine Gangbestimmungslogik speichert;

worin der Motor (153) die sich bewegenden Plattformen (159) antreibt, um das linke Pedal (161a) und das rechte Pedal (161b) in die entgegengesetzte Richtung zu bewegen; wobei der Motor (153) am Wendepunkt der Hin- und Herbewegung jeder der sich bewegenden Plattformen (159) in die entgegengesetzte Richtung gedreht wird, und zu diesem Zeitpunkt die Antriebsschaltung (151) ein Lenksignal erzeugt; wobei, wenn das linke Pedal (161a) und das rechte Pedal (161b) den Positionssensor (163) passieren, der Positionssensor (163) ein Durchgangssignal erzeugt;

worin die Gangerkennungseinheit (171a) Gewichtssignale, die von den Drucksensoren (162) oder Lastmesszellen oder Gyroskopen erfasst werden, das von dem Positionssensor (163) erfasste Durchgangssignal und das von der Antriebsschaltung (151) gelieferte Lenksignal empfängt; die Gangbeurteilungseinheit (171b) die Gewichtssignale, das Durchgangssignal und das Lenksignal von der Gangerkennungseinheit (171a) empfängt und die Schwerpunktsberechnungslogik verwendet, um das aufgelastete Gewicht des linken Pedals (161a) und des rechten Pedals (161b) und die Position des Schwerpunkts auf dem linken Pedal (161a) und dem rechten Pedal (161b) zu berechnen, und auch die Gangbestimmungslogik verwendet, um zu bestimmen, ob das aufgelastete Gewicht des linken Pedals (161a) und des rechten Pedals (161b) korrekt ist und ob die Position des Schwerpunkts des linken Pedals (161a) und des rechten Pedals (161b) korrekt ist.

2. Gangtrainingsmaschine (10) nach Anspruch 1, wobei die Gangbestimmungseinheit die Anzahl der Durchgänge, die Anzahl der Lenkzeiten, die Anzahl der Zeiten, die eine erste Bedingung erfüllen, die anzeigt, dass die Position des Schwerpunkts des linken Pedals (161a) und des rechten Pedals (161b) korrekt ist, und die Anzahl der Zeiten, die eine zweite Bedingung erfüllen, die anzeigt, dass die Knöchelbewegung des linken Fußes des Benutzers und des rechten Fußes korrekt ist, berechnet; wobei, wenn die Gangbestimmungseinheit das Durchgangssignal empfängt, die Anzahl der Durchgänge um eins erhöht wird, und die Gangbestimmungslogik verwendet wird, um zu bestimmen, ob das aufgelastete Gewicht des linken Pedals (161a) und des rechten Pedals (161b) korrekt ist, wobei, wenn es korrekt ist, die Anzahl der Male, die die erste Bedingung erfüllen, um eins erhöht wird, und wenn nicht, die Anzahl der Male, die die erste Bedingung erfüllen, unverändert bleibt; wobei, wenn die Gangbeurteilungseinheit (171b) das Lenksignal empfängt, die Anzahl der Lenkvorgänge um eins erhöht wird, und die Gangbestimmungslogik auch verwendet wird, um zu bestimmen, ob die Knöchelbewegung des linken Fußes und des rechten Fußes des Benutzers korrekt ist, wobei, wenn sie korrekt ist, die Anzahl der Vorgänge, die die zweite Bedingung erfüllen, um eins erhöht wird, und wenn nicht, die Anzahl der Vorgänge, die die zweite Bedingung erfüllen, unverändert bleibt.

3. Gangtrainingsmaschine (10) nach Anspruch 2, welche ferner mit einem an der Steuerung (17) angeordneten Auswertemodul (172), das eine Führungseinheit (172a) aufweist; wobei, nachdem die Führungseinheit (172a) das von der Antriebsschaltung (151) erzeugte Steuersignal empfängt, die Führungseinheit (172a) einen ersten Führungsindikator aussendet, um den Benutzer anzuleiten, den Schwerpunkt zu einem der Füße zu bewegen; und nachdem die Führungseinheit (172a) das Lenksignal erneut empfängt, die Führungseinheit (172a) einen zweiten Führungsindikator aussendet, um den Benutzer anzuleiten, den Schwerpunkt zum anderen Fuß zu bewegen.

**4.** Gangtrainingsmaschine (10) nach Anspruch 3, worin das Auswertemodul (172) ferner eine *Level-of Involvement*-Auswertungseinheit (172b) umfasst, die eine Knöchelgelenk-Korrekturrate und eine Schwerpunkt-Korrekturrate berechnet, worin die Knöchelgelenk-Korrekturrate = die Anzahl der Male, die die zweite Bedingung erfüllen / die Anzahl der Male des Lenkens * 100% ist, und die Schwerpunkt-Korrekturrate = die Anzahl der Male, die die erste Bedingung erfüllen / die Anzahl der Durchgänge * 100% ist.

**Revendications**

**1.** Machine d'entraînement à la marche (10), comprenant une base (11), ladite machine d'entraînement à la marche (10) comprenant en outre:

un module d'entraînement (15) disposé dans ladite base (11), ledit module d'entraînement (15) comprenant un circuit de commande (151), un moteur (153) et deux plates-formes mobiles (159), ledit circuit de commande (151) commandant ledit moteur (153) pour déplacer lesdites plates-formes mobiles (159);
un module de capteurs (16) comprenant une pédale gauche (161a), une pédale droite (161b), plusieurs capteurs de pression (162) ou cellules de charge ou gyroscopes, et un capteur de position (163), ladite pédale gauche (161a) et ladite pédale droite (161b) étant respectivement reliées de manière pivotante aux plates-formes mobiles (159), lesdits capteurs de pression (162) ou cellules de charge ou gyroscopes, étant respectivement montés sur ladite pédale gauche (161a) et ladite pédale droite (161b), ledit capteur de position (163) étant disposé dans ladite base (11);
un contrôleur (17) ayant pour fonction de stocker, de traiter et d'émettre des données, ledit contrôleur (17) étant connecté électriquement audit circuit de commande (151), à chaque capteur de pression (162) ou cellule de charge ou gyroscope, et audit capteur de position (163);
un module de traitement des signaux (171) disposé dans ledit contrôleur (17), ledit module de traitement des signaux (171) comprenant une unité de détection de la marche (171a) et une unité d'évaluation de la marche (171b), ladite unité d'évaluation de la marche (171b) stockant une logique de calcul du centre de gravité et une logique de détermination de la marche;
dans lequel ledit moteur (153) entraîne lesdites plates-formes mobiles (159) pour déplacer ladite pédale gauche (161a) et ladite pédale droite (161b) dans la direction opposée; au point d'inflexion du mouvement alternatif de chacune desdites plates-formes mobiles (159), ledit moteur (153) est tourné en sens inverse, et à ce moment, ledit circuit de commande (151) génère un signal de direction; lorsque ladite pédale gauche (161a) et ladite pédale droite (161b) passent devant ledit capteur de position (163), ledit capteur de position (163) génère un signal de dépassement;
dans lequel l'unité de détection de la marche (171a) reçoit les signaux de poids détectés par les capteurs de pression (162), les cellules de charge ou les gyroscopes, le signal de passage détecté par le capteur de position (163) et le signal de direction fourni par le circuit de commande (151); ladite unité d'évaluation de la marche (171b) reçoit lesdits signaux de poids, ledit signal de passage et ledit signal de direction de ladite unité de détection de la marche (171a), et utilise ladite logique de calcul du centre de gravité pour calculer le poids en appui de ladite pédale gauche (161a) et de ladite pédale droite (161b) et la position du centre de gravité sur ladite pédale gauche (161a) et ladite pédale droite (161b), et utilise également cette logique de détermination de la marche pour déterminer si le poids de la pédale gauche (161a) et de la pédale droite (161b) est correct et si la position du centre de gravité de la pédale gauche (161a) et de la pédale droite (161b) est correcte.

**2.** Machine d'entraînement à la marche (10) selon la revendication 1, dans lequel ladite unité de détermination de la marche calcule le nombre de passages, le nombre de temps de direction, le nombre de temps qui répondent à une première condition indiquant que la position du centre de gravité de ladite pédale gauche (161a) et de ladite pédale droite (161b) est correcte, et le nombre de temps qui répondent à une deuxième condition indiquant que le mouvement du pied gauche et de la cheville du pied droit de l'utilisateur sont corrects; lorsque l'unité de détermination de la marche reçoit le signal de passage, le nombre de passages est augmenté d'une unité et la logique de détermination de la marche est utilisée pour déterminer si le poids de la pédale gauche (161a) et de la pédale droite (161b) est correct; si c'est le cas, le nombre de fois où la première condition est remplie est augmenté d'une unité, sinon le nombre de fois où la première condition est remplie reste inchangé; lorsque l'unité d'évaluation de la marche (171b) reçoit le signal de direction, le nombre de fois où la direction est activée est augmenté d'une unité, et la logique de détermination de la marche est également utilisée pour déterminer si le mouvement de la cheville du pied gauche et du pied droit de l'utilisateur est correct; s'il est correct, le nombre de fois où ladite deuxième condition est remplie est augmenté d'une unité, sinon, le nombre de fois où ladite deuxième condition est remplie reste inchangé.

3. Machine d'entraînement à la marche (10) selon la revendication 2, comprenant en outre un module d'évaluation (172) disposé sur ledit contrôleur (17), ledit module d'évaluation (172) comprenant une unité de guidage (172a); après que ladite unité de guidage (172a) a reçu ledit signal de direction généré par ledit circuit de commande (151), ladite unité de guidage (172a) transmet un premier indicateur de guidage pour guider l'utilisateur afin de déplacer le centre de gravité vers l'un des pieds; après que ladite unité de guidage (172a) a reçu à nouveau ledit signal de direction, ladite unité de guidage (172a) transmet un deuxième indicateur de guidage pour guider l'utilisateur afin qu'il déplace le centre de gravité vers l'autre pied.

4. Machine d'entraînement à la marche (10) selon la revendication 3, dans laquelle ledit module d'évaluation (172) comprend en outre une unité d'évaluation du niveau d'implication (172b) qui calcule un taux de correction de l'articulation de la cheville et un taux de correction du centre de gravité, le taux de correction de l'articulation de la cheville = le nombre de fois qui remplissent ladite deuxième condition / le nombre de fois de direction * 100 %, et le taux de correction du centre de gravité = le nombre de fois qui remplissent ladite première condition / le nombre de passages * 100 %.

FIG. 1

FIG. 2

FIG. 3

FIG. 4

FIG. 5

EP 3 785 687 B1

Start

A)Preparation stage

B)Gait training machine action stage

C) Signal detection and judgment

D)Guiding display

E) Repeated action:
Training time ended?

No

Yes

End

FIG. 6

the center-of-gravity coordinate position of the left foot

FIG. 7

the center-of-gravity coordinate position of the right foot

FIG. 8

EP 3 785 687 B1

FIG. 9

FIG. 10

FIG. 11

FIG. 12

Start

↓

A)Preparation stage

↓

B)Gait training machine action stage

↓

C)Signal detection and judgment

↓

D)Guiding display

↓

E)Repeated action:
Training time ended? ──No──┐

Yes

↓

F) Result display

↓

End

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7266424 B2
- JP 2018102842 A
- WO 2007058512 A1
- US 10022587 B1